# EUROPEAN PATENT SPECIFICATION

(11) **EP 3 641 709 B1**
(45) Date of publication and mention of the grant of the patent: **20.03.2024**
(21) Application number: 18734968.3
(22) Date of filing: 11.06.2018
(51) Int. Cl.: A61F 9/007

(54) **ELECTRONICALLY ACTUATED RECIPROCATING SURGICAL INSTRUMENT**
ELEKTRONISCH BETÄTIGTES, HIN- UND HERGEHENDES CHIRURGISCHES INSTRUMENT
INSTRUMENT CHIRURGICAL À MOUVEMENT ALTERNATIF ACTIONNÉ ÉLECTRONIQUEMENT

(30) Priority: 21.06.2017 US 201762522974 P
(43) Date of publication of application: 29.04.2020
(73) Proprietor: ALCON INC., 1701 Fribourg (CH)
(72) Inventor: UNDERWOOD, John R., Lake Forest, CA 92630 (US)
(74) Representative: Bohest AG
(86) International application number: PCT/IB2018/054215
(87) International publication number: WO 2018/234924

(56) References cited:
- EP-A1- 3 038 574
- CN-B- 101 829 790
- US-A1- 2013 144 317
- US-A1- 2016 120 697
- US-A1- 2017 027 753
- US-B1- 6 575 990

## Description

### TECHNICAL FIELD

The present disclosure relates to ophthalmic surgery, and more specifically, to an electronically actuated reciprocating surgical instrument.

### BACKGROUND

Ophthalmic surgery is performed on the eye to save and improve the vision of tens of thousands of patients every year. However, given the sensitivity of vision to even small changes in the eye and the minute and delicate nature of many eye structures, ophthalmic surgery is difficult to perform and the reduction of even minor or uncommon surgical errors or modest improvements in precision or accuracy of surgical techniques can make a significant difference in the patient's vision after the surgery.

Ophthalmic surgical procedures are performed on an eye. Vitreoretinal surgical procedures are a class of ophthalmic surgical procedures that encompasses various delicate procedures involving internal portions of the eye, such as a vitreous humor and the retina. Vitreoretinal surgical procedures are performed, sometimes with lasers, to improve visual sensory performance. Vitreoretinal surgical procedures may be performed in the treatment of many eye diseases, including epimacular membranes; diabetic retinopathy; vitreous hemorrhage; macular hole; detached retina; complications of cataract surgery; or other eye diseases.

During vitreoretinal surgery, an ophthalmologist typically uses a surgical microscope to view a fundus through a cornea, while surgical instruments that penetrate a sclera may be introduced to perform any of a variety of different procedures. A surgical microscope provides imaging and optionally illumination of the fundus during vitreoretinal surgery. A patient typically lies supine under a surgical microscope during vitreoretinal surgery and a speculum is used to keep an eye exposed.

Modern ophthalmic surgery, such as vitreoretinal surgery, is typically performed with complex equipment, such as specialized surgical instruments; infusion pumps; pneumatic valves; pneumatic pumps; pneumatic compressors; aspirators; illumination sources; cooling fans; lasers; or other types of complex equipment. An example surgical instrument used in vitreoretinal surgeries is a handheld vitrectomy probe. In some instances, a vitrectomy probe is a reciprocating surgical instrument that uses dual pneumatic actuation inputs to control a duty-cycle of a reciprocating cutter.

Document EP 3038 574 is considered to represent the most relevant prior art and discloses the features of the preamble of claim 1.

### SUMMARY

The invention is defined in independent claim 1. The moveable cutter element may be fixed to the permanent magnet, and the electromagnetic coil may be fixed to the housing body. The moveable cutter element may be fixed to the electromagnetic coil and the permanent magnet is fixed to the housing body. The housing body may form a handle sized and shaped to be held in a hand of a user. magnetic drive unit includes a voice coil actuator. Electrical power may be supplied to the at least one electromagnetic coil from an external power source. Electrical power may be supplied to the at least one electromagnetic coil from a battery disposed in the housing body. The cutter may also include an outer cutter element, and the inner cutter element may be reciprocable within the outer cutter element.

Preferred embodiments may also include one or more of the following features. The reciprocating surgical instrument may be a handheld surgical instrument. The reciprocating surgical instrument is a vitrectomy probe. magnetic drive unit includes a voice coil actuator. Electrical power for energizing the electromagnetic coil may be supplied from an external power source. The electrical power for energizing the electromagnetic coil may be supplied from a battery disposed within the housing body. The cutter element may be a multi-cutter element.

### BRIEF DESCRIPTION OF THE DRAWINGS

For a more complete understanding of the present disclosure and the associated features and advantages described herein, reference is now made to the following description, taken in conjunction with the accompanying drawings, which may not be drawn to scale and in which like numerals refer to like features.
FIG. 1 shows a surgeon performing an ophthalmic surgery on an eye of a patient using an electronically actuated reciprocating surgical instrument;
FIG. 2A is a partial cross-sectional view of an example electronically actuated reciprocating surgical instrument;
FIG. 2B is a detail view of a partial cross-sectional of an example electronically actuated reciprocating surgical instrument; and
FIGs. 3A-3C show a distal end of an example electronically actuated reciprocating surgical instrument illustrating a port formed in a cutter of the reciprocating surgical instrument.

### DETAILED DESCRIPTION

For the purposes of promoting an understanding of the principles of the present disclosure, reference will now be made to the implementations illustrated in the drawings, and specific language will be used to describe the same.

Throughout this disclosure, a hyphenated form of a reference numeral refers to a specific instance of an element and the un-hyphenated form of the reference numeral refers to the element generically or collectively. Thus, as an example (not shown in the drawings), device "12-1" refers to an instance of a device class, which may be referred to collectively as devices "12" and any one of which may be referred to generically as a device "12". In the figures and the description, like numerals are intended to represent like elements.

A reciprocating surgical instrument may be a handheld surgical instrument that operates at relatively high cutting rates. For example, ULTRAVIT^{®} vitrectomy probes produced by Alcon Laboratories, Inc., located at 6100-2 South Freeway, Fort Worth, Texas 76134, are used during ophthalmic surgery for highly delicate and precise manipulations a surgeon. Vitrectomy probes typically include a cutter. The cutter includes a hollow outer cutter element, and a hollow inner cutter element arranged coaxially with each other. The inner cutter element moves within the outer cutter element in a reciprocating manner. The outer cutter element includes a cutting interface at a port. The port extends radially through the outer cutter element near a distal end thereof. Tissue is drawn into the port in response to an underpressure (referred to hereinafter as a vacuum) generated in the inner cutter element, and, when the inner cutter element is actuated to move distally towards the port, a cutting surface formed on the distal end of the inner cutter element, in cooperation with the cutting interface of the port, severs the tissue as the port closes. The tissue is then aspirated away through the inner cutter element. Tissue and other biological material that the vitrectomy probe removes, such as vitreous humor and retinal membranes, can vary in size.

Pneumatic actuation has been used to drive reciprocating surgical instruments. In the case of a single action pneumatic instrument, pneumatic pressure is utilized to displace the inner cutter element in a first direction, while a mechanical return mechanism, such as a mechanical spring, returns the inner cutter element in a second direction, opposite the first direction, thereby generating a reciprocating cutting action. In the case of a dual action pneumatic instrument, pneumatic pressure is utilized to actuate an inner cutter element in opposing first and second direction. However, pneumatically actuated surgical instruments may not have positional control of an actuated component.

The reciprocating surgical instruments disclosed herein contain an electronically actuated reciprocating component, such as, a reciprocating vitrectomy cutter. The reciprocating surgical instruments disclosed herein includes a magnet-coil actuation system, such as a voice coil actuator (VCA), that is operable to reciprocate the reciprocating surgical instrument. These electronically actuated reciprocating surgical instruments include a microsurgical cutting probe for posterior segment ophthalmic surgery, during which a surgeon can control the port size as desired. The size of the instrument's port may be adjusted to maximize cutting efficiency and tissue stability. The electronically actuated reciprocating surgical instruments disclosed herein may operate without external pneumatic actuators and compressors, which may provide greater simplicity and fewer sources of ambient acoustical noise in an operating room environment.

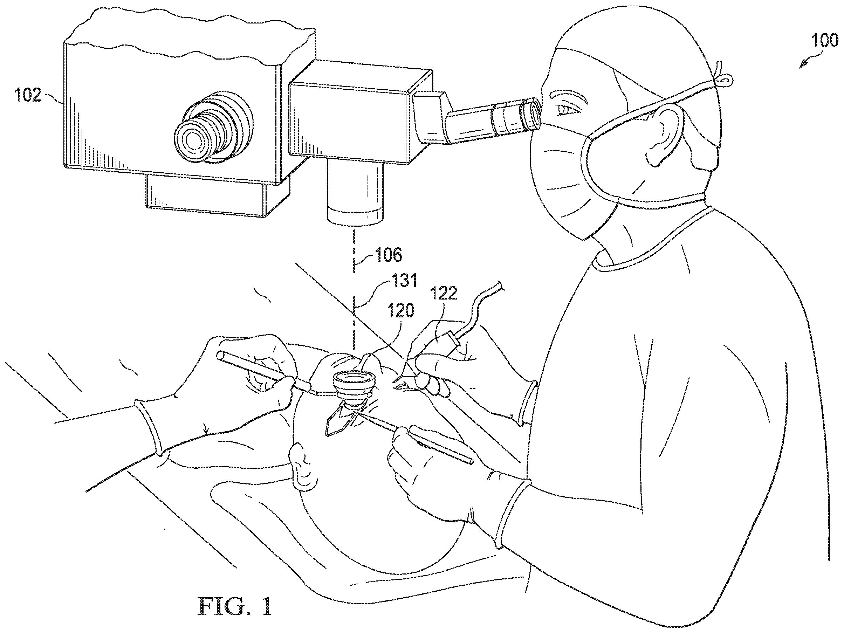

FIG. 1 illustrates an ophthalmic surgical procedure in which a surgical instrument 100 is utilized. In some instances, the surgical instrument 100 may be a reciprocating surgical instrument. FIG. 1 shows a surgeon 120 performing an ophthalmic surgery on an eye 104 of a patient 130 using the surgical instrument 100. The surgical instrument 100 is electronically actuated. As shown in FIG. 1, the eye 104 is exposed using a speculum 140, and a contact lens 150 is held in place on the eye 104 and is visually aligned with a surgical microscope 102 to facilitate visualization of inner structures of the eye 104. The surgeon 120 uses the surgical instrument 100 to perform surgery on structures of the eye 104, such as, for example, removal of vitreous humor from the posterior segment of the eye 104.

For example, in some instances, the surgical instrument 100 may be a vitrectomy probe. The surgeon 120 uses the surgical instrument 100 to remove the clear, gel-like vitreous humor ("vitreous") that normally fills the posterior segment of the eye 104. The surgeon removes vitreous while avoiding interaction with nearby eye structures, such as the retina, that are extremely sensitive to physical contact. The surgeon 120 may attempt to remove the vitreous from the eye 104 as quickly as possible in order to limit exposure of the retina to the light used to visualize the vitreous and, thereby, reduce potential injury to the retina that may result from excessive light levels. Surgical operations associated with the use of the surgical instrument 100 may involve sub-millimeter precision corresponding to fine biostructures in an eye, resulting in very small dimensional tolerances under which surgical instrument 100 operates.

As shown in FIG. 1, the surgical instrument 100 is an electronically actuated instrument. An electrical connection, shown as a cable 110, extends from the surgical instrument 100 to a power supply, which, in some instances, may be included in a surgical console, such as a Constellation^{®} Vision System produced by Alcon at 6201 South Freeway, Fort Worth, Texas. The cable 110 supplies electrical power to the surgical instrument 100. The electrical power may be, for example, a 24 volt direct current. In other instances, the electrical power may be an alternating current or direct current at any desired voltage and current. In other implementations, instead of an external source of electrical power, the surgical instrument 100 may include an internal power supply, such as a battery (not shown), that may provide electrical power for the surgical instrument 100. The battery may be disposed in the housing body of the surgical instrument 100. Thus, in some implementations, the cable 110 may be omitted.

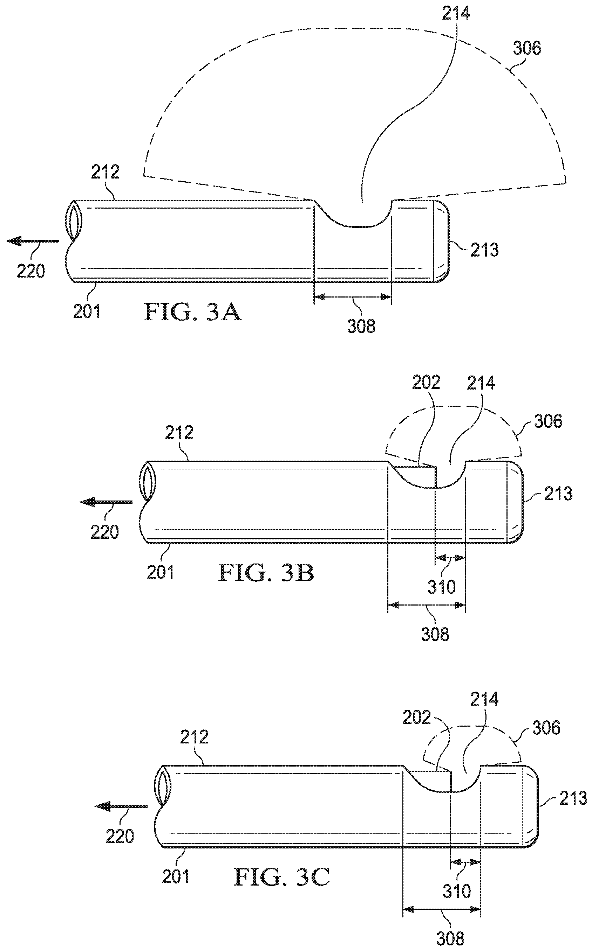

In contrast to a pneumatically driven surgical instrument, electronic actuation of the surgical instrument 100 provides the ability to positionally control (i.e., control the position of and, particularly, the fully retracted position of) the reciprocating component of the surgical instrument 100. In the instance where the surgical instrument 100 is a vitrectomy probe, the electronically actuated reciprocating component may be a cutting element. For example, in the context of a vitrectomy probe, a proximal-most position of an inner cutter element along a longitudinal axis of the vitrectomy probe may be selected and controlled during actuation of inner cutter element in order to vary the opening size of the cutter port. With the ability to vary the opening size of the cutter port, a region of space proximate to the cutter port on which a vacuum applied through the cutter port influences surrounding material (i.e., a region of influence) may be altered. For example, with a decreased port size, the region of influence is also decreased. With an increased port size, the region of influence similarly increases, as shown, for example, in FIGs. 3A-3C. Without positional control of the reciprocating component, such as the inner cutting element of a vitrectomy probe, the surgeon 120 may have limited control, or a limited range of control, of the performance of the surgical instrument 100. For example, for surgical instruments lacking the ability to positionally control the reciprocating component, the ability of the surgeon to precisely cut a desired amount of tissue by limiting the region of influence at the port of the surgical instrument might be constrained due to the lack of positional control of the cutting element. In such instances, control of such a surgical instrument may be limited to the geometric construction of the surgical instrument, e.g., a size of the opening of the port.

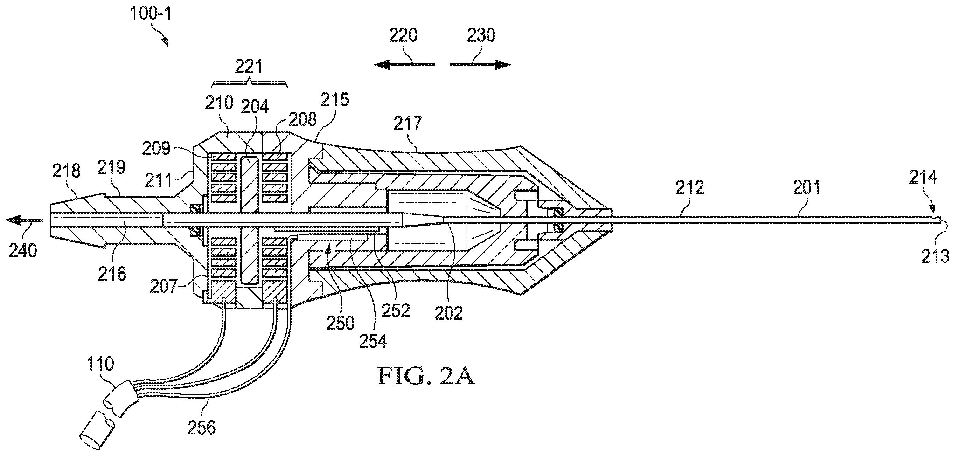

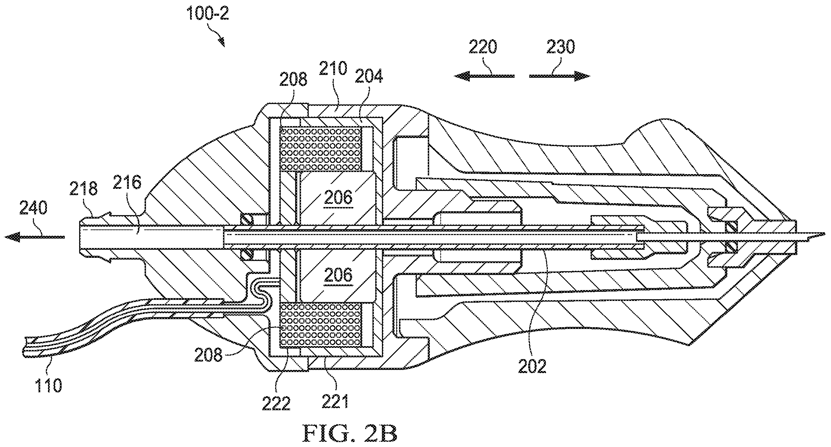

The surgical instrument 100 contains a magnetic drive unit that includes a permanent magnet and one or more electromagnetic coils (see also FIGs. 2A and 2B). The magnetic drive unit may be arranged in any of a variety of configurations in different implementations. For example, the permanent magnet may be fixed, while the electromagnetic coil may be attached to a cutting element that is reciprocable, i.e., the cutting element is operable to reciprocate. In other examples, the electromagnetic coil may be fixed, while the permanent magnet may be attached to a cutting element that is reciprocable. In some implementations, the magnetic drive unit is a (VCA) that contains one or more positional feedback sensors, thereby enabling precise positional control of a cutting element. The one or more positional feedback sensors may also be included with the magnetic drive unit, when the magnetic drive unit is not a VCA. In this manner, the surgical instrument 100 may enable a user, such as a surgeon, to achieve very fine control of the surgical operations performed using the surgical instrument 100, including very fine control, such as control of the size of a port formed in a vitrectomy probe. The size of the port may be defined by a position of a cutting element that is reciprocated in response to actuation of the magnetic drive unit.

FIG. 2A is a cross-sectional view of an example surgical instrument 100-1 that is in the form of a vitrectomy probe that includes a reciprocating cutting element. The surgical instrument 100-1 is electronically actuated. The surgical instrument 100-1 may represent the surgical instrument 100 shown in FIG. 1. The surgical instrument 100-1 includes a housing body 210, a magnet 204, two electrical coils 208 and 209, and a cutter 201. The cutter 201 extends from a distal end of the housing body 210 and includes an inner cutter element 202 and an outer cutter element 212. The inner cutter element 202 is a tubular member that has open proximal and distal ends and defines a passage therethrough. The outer cutter element 212 defines an inner passage and has a closed distal end 213. The inner cutter element 202 is received within the passage of the outer cutter element 212 and is slideable therein in opposing directions. A port 214 is formed in the outer cutter element 212 at a distal end thereof. The surgical instrument 100-1 also includes a power cable 110 that provides electrical power to the electrical coils 208, 209 and a connector 218 formed at proximal end 219 of the housing body 210 for application of a vacuum. In other implementations, the surgical instrument 100-1 may be implemented with fewer or more components.

As depicted in FIG. 2A, the housing body 210 may be formed of two or more sections. Particularly, in the implementation shown in FIG. 2A, the housing body 210 is formed from three connected sections 211, 215, and 217. In other implementations, the housing body 210 may be formed with fewer or more sections than shown in FIG. 2A.

As explained above, the surgical instrument 100-1 contains a magnetic drive unit 221 that includes a magnet 204 and two electrical coils 208 and 209. The housing 210 defines a chamber 207, and the magnetic drive unit is disposed in the chamber 207. The two electrical coils 208 and 209 are disposed on either side of the magnet 204. Particularly, a first electrical coil 208 is disposed proximally relative to the magnet 204, and a second electrical coil 209 is disposed distally relative to the magnet 204. In some implementations, such as the implementation illustrated in FIG. 2A, the magnet 204 may be fixed to the inner cutter element 202. As explained above, the inner cutter element 202 is a hollow, tubular member that reciprocates within a hollow outer cutter element 212. In various implementations, the port 214 may be a small fixed opening. In some instances, the port 214 may have a shape that is square, rectangular, oval, or any other desired shape. Further, in some instances, corners of the port may be rounded. In some instances, a dimension of the port 214 that is transverse to an axis extending longitudinally along the cutter 201 may be less than 1 mm wide, less than 500 µm wide, or about 350 µm wide. However, in other implementations, the width of the port 214 may be larger or smaller than the sizes indicated. Further, the port 214 may be any desired size or shape, which may, for example, depend of the application of the surgical instrument 100-1. In various implementations, the outer cutter element 212 may be formed using a 20 gage tube; a 22 gage tube; a 23 gage tube; a 25 gage tube; or a 27 gage tube. However, the scope of the disclosure is not so limited. Rather, the size of the outer cutter element 212 may be any desired size.

The inner cutter element 202 reciprocates within the outer cutter element 212 in the directions of arrows 230 and 220 in response to actuation of the magnetic drive unit 221. The inner cutter element 202 includes a distal tip, which, for example, may be circular in cross-section. The distal tip is configured to cooperate with the port 214 to sever material extending through the port 214. Particularly, the distal tip of the inner cutter element 202 may cooperate with a distal edge of the port 214 to sever material extending through the port 214. In some instances, a port may be formed in the distal end of the inner cutter element 202 such that the cutter 201 performs two cuts per cycle of the inner cutter 202. Movement of the inner cutter element 202 in the direction of arrow 230 causes the distal tip thereof to move across the port 214. When the inner cutter element 202 has fully extended in the direction of arrow 230, the port 214 is in a closed condition. During this motion of the inner cutter element 202, any material extending into the port 214, such as, for example, vitreous, is cut. Movement of the inner cutter element 202 in the direction of arrow 220 retracts the inner cutter element 202, placing the port 214 in an open condition.

In some implementations, the magnetic drive unit 221 may include one or more position sensors. As shown in FIG. 2A, the surgical instrument 100-1 includes a position sensor 250. In the illustrated example, the position sensor 250 includes a first sensor component 252 coupled to and movable with the inner cutter element 202 and a second sensor component 254 fixed to the housing body 210. The position sensor 250 is operable to detect a position of the inner cutter element 202 by a position of the first sensor component 252 relative to the second sensor component 254. Positional information is transmitted along a one or more wires 256 to a controller, such as a controller included in a surgical console. In other instances, the controller may be provided separate from a surgical console. Although a single position sensor 250 is shown in FIG. 2A, other implementations may include more than one position sensor. The controller is operable to control a position of the inner cutter element 202 relative to the outer cutter element 212. As a result, the controller is operable to control a position of a distal end of the inner cutter element 202 relative to the port 214 to control a size of the port 214, as discussed below.

The one or more position sensors are used to controlling a position of the inner cutter element 202. For example, the position sensors may be used to control where the distal tip of the inner cutter element 202 is positioned relative to the port 214 when extension or retraction of the inner cutter element 202 is stopped. That is, the position sensors may be used to control a position of the distal tip of the inner cutter element 202 relative to the port 214 when the inner cutter element 202 is at an extended or retracted position. For example, in some instances, the position sensors may be used to place the inner cutter element 202 in a partially retracted position such that the distal tip thereof is located at a position between the proximal and distal edges of the port 214. Thus, in such instances, with the inner cutter element 202 in a partially retracted position, the inner cutter element 202 obstructs a proximal portion of the port 214 resulting in an opening of the port 214 is less than the full size of the port 214. A position of the inner cutter element 202 may be controlled to partially open the port 214 to any desired opening size, thereby enabling precisely controlled variation of the size of the port 214. Consequently, vitrectomy instruments within the scope of the present disclosure provide for varying the opening size of the port 214 during a surgical procedure, unlike conventional vitrectomy probes that include no such capability or functionality.

At the proximal end of the housing body 210, the connector 218 provides for a connection to an aspiration line. A vacuum applied to the aspiration line operates to transport material through a hollow channel 216 extending through the housing body 210 and in fluid communication with the inner passage formed in the inner cutter element 202 and the passage formed in the outer cutter element 212. Accordingly, the outer cutter element 212 is fluidically coupled to the inner cutter element 202, which is, in turn, fluidically coupled to the connector 218. Aspirated materials are conveyed along this combined passage and are removed from the surgical instrument 100-1. The aspirated materials are transported away through the aspiration line connected to the connector 218. In this manner, the tissue, material that is cut at the port 214, and other materials may be removed during operation of the surgical instrument 100-1. Furthermore, with ability to control the size of the port 214 in combination with the ability to control an amount of vacuum applied at the connector 218, the surgical instrument 100-1 enables precise control of removal of material within a region surrounding the port 214, i.e., the region of influence, illustrated in FIGs. 3A, 3B, and 3C. As explained above, the region of influence is a region proximate to the port 214 in which the applied vacuum will influence surrounding material. In particular, a reduction of the size of the port 214, as described above, may provide the surgical instrument 100-1 with a small region of influence to give the user a higher degree of precision that enables removing a smaller amount of material than would be possible using conventional surgical instruments without positional control of the inner cutter element 202. Thus, by having the ability to control the size of the port 214, a user, e.g., a surgeon, is able to target smaller areas and make smaller, more precise cuts that are otherwise unavailable where the port 214 is not adjustable.

Thus, as the inner cutter element 202 extends in the first direction 230, the port 214 is fully closed and the material that has been drawn through the port 214 and into the outer cutting element 212 is cut as the distal tip of the inner cutting element 202 slides across the port 214. In some implementations, an edge of the outer cutter element 212 that defines the port 214 may be sharpened so as to cooperate with the distal tip of the inner cutter element 202 to perform a cutting action.

In further implementations and as explained above, the inner cutting element 202 may include a second port, which is referred to herein as a "multi-cutter element". As the inner cutting element 202 extends in the direction of arrow 230 across the port 214, the second port formed in the inner cutting element 202 may become aligned with the port 214, thereby enabling material to be drawn into the cutter 201 through the overlapping ports formed in the inner cutter element 202 and the outer cutter element 212. The ingested material is severed as the inner cutting element 202 retracts in the second direction arrow 220. In this manner, the surgical instrument 100-1 produces multiple cuts for each reciprocation of the inner cutter element 202.

During operation of the example magnetic drive unit 221 shown in FIG. 2A, the two electromagnetic coils 208 and 209 are alternatingly activated to alternatingly attract the magnet 204 in the direction of arrows 220 and 230, respectively. In this manner, the magnet 204 that is coupled to the inner cutter element 202 drives the inner cutter element 202 in a reciprocating fashion. The power cable 110 supplies electrical power to the electromagnetic coils 208 to activate the magnetic drive unit 221 for the reciprocating motion. As shown, the power cable 110 includes two electrical lines. However, in other implementations, the power cable 110 may include additional electrical lines. For example, in other implementations, the power cable 110 may include one or more additional electrical lines to carry a positional feedback signal from the magnetic drive unit 221. The positional feedback signals may be provided to a controller (e.g., a microprocessor having access to memory media storing instructions executable by the microprocessor), and the controller may user the positional feedback signal information to control a position of the inner cutter element 202. One or more programs, e.g., software instructions, executed by the controller permits the controller to control a position of the inner cutter element 202. In some implementations, the two electrical lines in the power cable 110, such as those shown in FIG. 2A, may also be used to carry the positional feedback signal, such as by using a modulation technique. The controller may form part of a console that is operable to control one or more operations of the surgical instrument 110-1. In various implementations, the surgical instrument 100-1 may operate at reciprocation rates of up to 1,000 cycles per second. In other instances, the rate of reciprocation may be within the range of 300 to 500 cycles per second. However, the scope of the disclosure is not so limited. Thus, the rate of reciprocation may be higher or lower than the indicated range. However, the scope of the disclosure is not so limited. Rather, in other implementations, a magnetic drive unit may have other configurations and may operate at different ranges of reciprocation rates.

FIG. 2B shows a detail view of a partial cross-section of another example electronically actuated reciprocating surgical instrument 100-2. The surgical instrument 100-2 may represent the surgical instrument 100 in FIG. 1. The reciprocating surgical instrument 100-2 is similar in many aspects and features as described above with respect to the reciprocating surgical instrument 100-1 in FIG. 2A. The surgical instrument 100-2 includes a housing body 210, a power cable 110, a magnetic drive unit 221, and a cutter similar to cutter 201 of the surgical instrument 100-1. The cutter 201 and the description thereof, included above, are applicable to surgical instrument 100-2. As such, the details of the cutter of surgical instrument 100-2 are not repeated here. Consequently, discussion of the cutter and the parts thereof associated with surgical instrument 100-2 may be referenced in FIG. 2A.

The magnetic drive unit 221 includes a voice coil actuator 222. The VCA 222 includes a magnet 206 housed in a magnet frame 204 such that the magnet 206 and the magnet frame 204 are fixed relative to the housing body 210 and do not move within the surgical instrument 100-1. In some instances, the magnetic frame 204 may be formed from a magnetically soft material that can be magnetized by the fixed magnet 206, such as a ferromagnetic material. However the disclosure is not so limited. Rather, the magnet frame 204 may be formed from any suitable material. For example, the magnetic frame 204 may be formed from any suitable magnetic material. The VCA 222 also includes an electromagnetic coil 208. The electromagnetic coil 208 is fixed relative to the inner cutter element 202. In some instances, the electromagnetic coil 208 may be fixed directly or indirectly to the inner cutter element 202. The electromagnetic coil 208 interacts with the magnet 206 to cause the electromagnetic coil 208 and the inner cutter element 202 to reciprocate within the housing body 210 and relative to the outer cutter element 212. In some instances, the VCA 222 further contains one or more position sensors that provide positional feedback of the inner cutter element 202 (as shown, for example, in FIG. 2A), which may be used to enable positional control of the inner cutter element 202 relative to the outer cutter element 212 during operation of the cutter 201 and, particularly, with respect to the distal tip of the inner cutter element 202 relative to the port 214.

The housing body 210 includes a connector 218. Similar to the connector 218 described above, aspiration tubing may be attached to the surgical instrument 100-2 at the connector 218. A vacuum may be applied to a hollow channel 216. The vacuum is operable cause materials to be aspirated from the eye. The power cable 110 supplies electrical power to the electromagnetic coil 208 to activate the VCA 222 and produce reciprocating motion of the inner cutter element 202. In some implementations, the power cable 110 may also carry a positional feedback signal from the VCA 222. As explained above in the context of the surgical instrument 100-1, the positional feedback signals may be provided to a controller. The controller may be a microprocessor having access to memory media storing instructions executable by the microprocessor and execute software instructions. As a result, the controller is operable to control a longitudinal position of the inner cutter element 202 relative to the inner cutter element 212. In some implementations, the two electrical lines in the power cable 110 may also be used to carry the positional feedback signal, such as using a modulation technique. In various implementations, the surgical instrument 100-2 may operate at reciprocation rates of up to 1,000 cycles per second. However, the scope of the disclosure is not so limited. Rather, in other implementations, a VCA may have other configurations and may operate at different ranges of reciprocation rates. The rate of reciprocation is within the range of 300 to 500 cycles per second. Still further, the rate of reciprocation may be higher or lower than the indicated range.

FIGs. 3A, 3B, and 3C illustrate a distal end of the cutter 201 shown in FIG. 2A and applicable as well to FIG. 2B. Specifically, FIGs. 3A, 3B, and 3C illustrate positional control of the inner cutter element 202 during reciprocation thereof and the resulting opening size of the port 214 as a result. Consequently, FIGs. 3A, 3B, and 3C illustrate aspects of the positional control of the inner cutter element 202 described previously to alter opening size of the port 214 and a region of influence 306 at the port 214. It is noted that the opening sizes 310 of the port 214, the sizes of the region of influence 306, and the applied vacuum described below are exemplary implementations for descriptive purposes. In other implementations, any number of opening sizes 310 of the port 214 may be created and any vacuum settings may be used.

In FIG. 3A, the port 214 is shown as being completely open, corresponding to the inner cutter element 202 (not visible in FIG. 3A) being in a fully retracted position in the first direction 220 during a reciprocating motion. With the inner cutter element 202 in the fully retracted position shown in FIG. 3A, an opening size of the port 214 is a full port width 308. In some implementations, the full port width 308 may be 350 µm. The region of influence 306 of the applied vacuum associated with fully opened port 214 extends outward to affect surrounding material in the eye 104, as shown in FIG. 3A. Further, the region of influence is largest for a fully opened port 214 for a selected applied vacuum. In the example shown in FIG. 3A, the region of influence 306 may correspond to a vacuum pressure of 600 mm Hg.

In FIG. 3B, the fully retracted position of the inner cutter element 202 is located positioned such that an opening size 310 of the port 214 is less than the full port width 308. In this example, the opening size 310 of the port 214 may be 100 µm, which is less than the full port width 308 of 350 µm. The corresponding region of influence 306 is reduced from that of FIG. 3A, and extends outwardly from the cutter 201 to a lesser amount.

In FIG. 3B, the region of influence 306 is shown for an vacuum pressure of 600 mm Hg, which corresponds to the same vacuum pressure associated with fully opened port 214 shown in FIG. 3A. As a result of the reduced opening size 310 of the port 214 shown in FIG. 3B, the region of influence 306 is smaller than that associated with FIG. 3A. Thus, by controlling a fully retracted position of the inner cutter element 202, a user, such as a surgeon, is able to more precisely control the region of influence and, as a result, cutting during a vitrectomy procedure.

In FIG. 3C, the port 214 is shown as being partially open as a result of adjustment of the fully retracted position of the inner cutter element 202. Similar to FIG. 3B, the fully retracted positon of the inner cutter element 202 is such that the distal end of the inner cutter element 202 is located between the proximal-most and the distal-most extent of the port 214. Thus, the opening size 310 is less than the full port width 308. In some implementations, the opening size 310 shown in FIG. 3C may be 100 µm, which is less than an example full port width 308 of 350 µm.

The opening size 310 of port 214 shown in FIG. 3C is the same as the opening size 310 of port 214 shown in FIG. 3B. However, the region of influence shown in FIG. 3C is smaller and extends a shorter distance away from the cutter 201 because the applied vacuum pressure is less than that shown in FIG. 3B. For example, the applied vacuum pressure associated with FIG. 3C may be 250 mm Hg, whereas the applied vacuum pressure associated with FIG. 3B may be 600 mm Hg. Similarly, the region of influence may be increased for a constant opening size 310 of the port 214 by increasing the applied vacuum pressure. Thus, with a constant opening size 310 maintained during reciprocation of the inner cutter element 202, the region of influence may be adjusted and more precisely controlled by changing the applied vacuum pressure.

Moreover, as is clear by the above-provided description, the region of influence may be controlled both by controlling a fully retracted position of the inner cutter element 202 (and, hence, a size of the opening 310 of the port 308) and by adjusting a vacuum pressure applied to the port 214.

As disclosed herein, a handheld reciprocating surgical instrument may be an electronically actuated reciprocating surgical instrument. The electronic actuation may be provided by a magnetic drive unit. The magnetic drive unit is a voice coil actuator (VCA). The magnetic drive unit may include positional feedback to enable positional control of a cutter element. In this manner, port-based flow control may be used to more precisely enable surgeons to accomplish surgical objectives and to control a size of the region of influence on mobile tissue affected by the cutter, for example.

## Claims

1. A reciprocating vitrectomy probe (100, 100-2) for use in ophthalmic surgery, comprising:
a housing body (210);
a cutter (201) extending from a distal end of the housing body (210), the cutter (201) comprising a moveable inner cutter element (202), wherein the cutter (201) further comprises an outer cutter element (212), and wherein the moveable inner cutter element (202) is reciprocable within the outer cutter element (212);
a magnetic drive unit (221) disposed in the housing body (210), the magnetic drive unit (221) comprising:
a permanent magnet (206); and
at least one electromagnetic coil (208), one of the permanent magnet (206) and the electromagnetic coil (208) being fixed to the housing body (210) and the other of the permanent magnet (206) and the electromagnetic coil (208) being fixed to the moveable inner cutter element (202) and the magnetic drive unit (221) operable to reciprocate the moveable inner cutter element (202),
**characterized in that** the magnetic drive unit (221) comprises a voice coil actuator (222) **and in that** the magnetic drive unit (221) is configured to reciprocate the moveable inner cutter element (202) at a rate within a range of 300 to 500 cycles per second.

2. The reciprocating vitrectomy probe of claim 1, wherein the moveable inner cutter element (202) is fixed to the permanent magnet (206) and the electromagnetic coil (208) is fixed to the housing body (210).

3. The reciprocating vitrectomy probe of claim 1, wherein the moveable inner cutter element (202) is fixed to the electromagnetic coil (208) and the permanent magnet (206) is fixed to the housing body (210).

4. The reciprocating vitrectomy probe of claim 1, wherein the housing body (210) forms a handle sized and shaped to be held in a hand of a user.

5. The reciprocating vitrectomy probe of claim 1, wherein electrical power is supplied to the at least one electromagnetic coil (208) from an external power source.

6. The reciprocating vitrectomy probe of claim 1, wherein electrical power is supplied to the at least one electromagnetic coil (208) from a battery disposed in the housing body (210).

7. The reciprocating vitrectomy probe of claim 1 further comprising a positional sensor (250) operable to detect a position of the movable inner cutter element (202) relative to the housing body (210).

## Patentansprüche

1. Hin- und hergehende Vitrektomiesonde (100, 100-2) zur Verwendung in der Augenchirurgie, umfassend:
einen Gehäusekörper (210),
ein Schneidwerkzeug (201), das sich von einem distalen Ende des Gehäusekörpers (210) erstreckt, wobei das Schneidwerkzeug (201) ein bewegliches inneres Schneidwerkzeugelement (202) umfasst, wobei das Schneidwerkzeug (201) ferner ein äußeres Schneidwerkzeugelement (212) umfasst und wobei das bewegliche innere Schneidwerkzeugelement (202) innerhalb des äußeren Schneidwerkzeugelements (212) hin- und herbewegbar ist;
eine Magnetansteuereinheit (221), die in dem Gehäusekörper (210) angeordnet ist, wobei die Magnetansteuereinheit (221) umfasst:
einen Permanentmagneten (206); und
wenigstens eine elektromagnetische Spule (208), wobei entweder der Permanentmagnet (206) oder die elektromagnetische Spule (208) am Gehäusekörper (210) befestigt ist und das jeweils andere von Permanentmagnet (206) bzw. elektromagnetischer Spule (208) an dem beweglichen inneren Schneidwerkzeugelement (202) befestigt ist, und die magnetische Antriebseinheit (221), die betriebsfähig ist, um das bewegliche innere Schneidwerkzeugelement (202) hin- und herzubewegen,
**dadurch gekennzeichnet, dass** die Magnetansteuereinheit (221) einen Schwingspulenbetätiger (222) umfasst,
und dadurch, dass die Magnetansteuereinheit (221) dafür ausgelegt ist, das bewegliche innere Schneidwerkzeugelement (202) mit einer Rate in einem Bereich von 300 bis 500 Zyklen pro Sekunde hin- und herzubewegen.

2. Hin- und hergehende Vitrektomiesonde nach Anspruch 1, wobei das bewegliche innere Schneidwerkzeugelement (202) am Permanentmagneten (206) befestigt ist und die elektromagnetische Spule (208) am Gehäusekörper (210) befestigt ist.

3. Hin- und hergehende Vitrektomiesonde nach Anspruch 1, wobei das bewegliche innere Schneidwerkzeugelement (202) an der elektromagnetischen Spule (208) befestigt ist und der Permanentmagnet (206) am Gehäusekörper (210) befestigt ist.

4. Hin- und hergehende Vitrektomiesonde nach Anspruch 1, wobei der Gehäusekörper (210) einen Griff bildet, der so bemessen und geformt ist, dass er von einem Benutzer in der Hand gehalten werden kann.

5. Hin- und hergehende Vitrektomiesonde nach Anspruch 1, wobei die wenigstens eine elektromagnetische Spule (208) von einer externen Stromquelle mit elektrischem Strom versorgt wird.

6. Hin- und hergehende Vitrektomiesonde nach Anspruch 1, wobei die wenigstens eine elektromagnetische Spule (208) von einer Batterie, die im Gehäusekörper (210) angeordnet ist, mit elektrischem Strom versorgt wird.

7. Hin- und hergehende Vitrektomiesonde nach Anspruch 1, die ferner einen Positionssensor (250) umfasst, der betriebsfähig ist, um eine Position des beweglichen inneren Schneidwerkzeugelements (202) relativ zum Gehäusekörper (210) zu erkennen.

## Revendications

1. Sonde de vitrectomie à mouvement alternatif (100, 100-2) destinée à être utilisée en chirurgie ophtalmique, comprenant :
un corps de boîtier (210) ;
un dispositif de coupe (201) s'étendant à partir d'une extrémité distale du corps de boîtier (210), le dispositif de coupe (201) comprenant un élément de coupe interne mobile (202), le dispositif de coupe (201) comprenant en outre un élément de coupe externe (212), et l'élément de coupe interne mobile (202) étant mobile en va-et-vient à l'intérieur de l'élément de coupe externe (212),
une unité d'entraînement magnétique (221) disposée dans le corps de boîtier (210), l'unité d'entraînement magnétique (221) comprenant :
un aimant permanent (206), et
au moins une bobine électromagnétique (208), l'un de l'aimant permanent (206) et de la bobine électromagnétique (208) étant fixé au corps de boîtier (210) et l'autre de l'aimant permanent (206) et de la bobine électromagnétique (208) étant fixé à l'élément de coupe interne mobile (202), et l'unité d'entraînement magnétique (221) pouvant actionner le mouvement de va-et-vient de l'élément de coupe interne mobile (202),
**caractérisée en ce que** l'unité d'entraînement magnétique (221) comprend un actionneur de bobine acoustique (222) et **en ce que** l'unité d'entraînement magnétique (221) est configurée pour faire aller et venir l'élément de coupe interne mobile (202) à une vitesse comprise entre 300 et 500 cycles par seconde.

2. Sonde de vitrectomie à mouvement alternatif selon la revendication 1, l'élément de coupe interne mobile (202) étant fixé à l'aimant permanent (206) et la bobine électromagnétique (208) étant fixée au corps de boîtier (210).

3. Sonde de vitrectomie à mouvement alternatif selon la revendication 1, l'élément de coupe interne mobile (202) étant fixé à la bobine électromagnétique (208) et l'aimant permanent (206) étant fixé au corps de boîtier (210).

4. Sonde de vitrectomie à mouvement alternatif selon la revendication 1, le corps de boîtier (210) formant une poignée dimensionnée et façonnée pour être tenue dans la main d'un utilisateur.

5. Sonde de vitrectomie à mouvement alternatif selon la revendication 1, l'énergie électrique étant fournie à au moins une bobine électromagnétique (208) à partir d'une source d'énergie externe.

6. Sonde de vitrectomie à mouvement alternatif selon la revendication 1, l'alimentation électrique de l'au moins une bobine électromagnétique (208) provenant d'une batterie disposée dans le corps de boîtier (210).

7. Sonde de vitrectomie à mouvement alternatif selon la revendication 1, comprenant en outre un capteur de position (250) capable de détecter une position de l'élément de coupe interne mobile (202) par rapport au corps de boîtier (210).
